Europäisches Patentamt

European Patent Office

Office européen des brevets

(11) Publication number: **0 297 585**
**A2**

(12) # EUROPEAN PATENT APPLICATION

(21) Application number: **88110488.9**

(22) Date of filing: **30.06.88**

(51) Int. Cl.4: **C12N 15/00 , C12P 21/02 , C12P 21/00 , C07K 13/00 , A61K 37/02 , A61K 39/395**

(30) Priority: **01.07.87 US 68917**

(43) Date of publication of application:
**04.01.89 Bulletin 89/01**

(84) Designated Contracting States:
**AT BE CH DE ES FR GB GR IT LI LU NL SE**

(71) Applicant: **Sloan-Kettering Institute For Cancer Research**
**1275 York Avenue**
**New York New York 10021(US)**

(72) Inventor: **Dropcho, Edward J.**
**3937 Briar Oak Drive**
**Birmingham Alabama 35243(US)**
Inventor: **Chen, Yao-Tseng**
**435 East 70th Street, Apt. 5K**
**New York New York 10021(US)**
Inventor: **Posner, Jerome B.**
**430 East 63rd Street**
**New York New York 10021(US)**
Inventor: **Old, Lloyd J.**
**600 West End Avenue**
**New York New York 10024(US)**

(74) Representative: **Henkel, Feiler, Hänzel & Partner**
**Möhlstrasse 37**
**D-8000 München 80(DE)**

(54) **Neuronal polypeptide and methods of production and use thereof.**

(57) This invention provides a neuronal polypeptide having a defined amino acid sequence. This invention also provides a nucleic acid molecule encoding the polypeptide. This invention further provides methods for producing the polypeptide. Finally, this invention provides the use of the polypeptide to prevent paraneoplastic cerebellar degeneration.

EP 0 297 585 A2

## NEURONAL POLYPEPTIDE AND METHODS OF PRODUCTION AND USE THEREOF

### Background of the Invention

This invention was made with government support under Grant Number CA-08748 from the National Cancer Institute. The U.S. Government has certain rights in the invention.

Throughout this application various publications are referenced by arabic numerals within parentheses. Full citations for these references may be found at the end of the specification immediately preceding the claims. The disclosures of these publications in their entireties are hereby incorporated by reference into this application in order to more fully describe the state of the art as known to those skilled therein as of the date of the invention described and claimed herein.

Several distinctive neurologic disorders of unknown etiology occur as "paraneoplastic syndromes" or "remote effects" of systemic cancer (1). The syndrome of paraneoplastic cerebellar degeneration (PCD) is among the most common of these disorders and generally occurs in patients with neoplasms of the lung (especially small cell carcinoma), breast, ovary, or with Hodgkin's disease (1, 2). Neuropathologic features of PCD include extensive loss of Purkinje cells, degenerative changes in the remaining Purkinje cells, variable loss of granule and basket neurons, proliferation of Bergmann glial cells, and a variable degree of perivascular and meningeal infiltration by lymphocytes and other mononuclear cells. The presence of anti-Purkinje cell antibodies in some PCD patients suggests an autoimmune etiology (3-6). Immunofluorescent staining of Purkinje cell cytoplasm has been reported in cases of PCD associated with Hodgkin's disease (3), small cell lung cancer (4), breast cancer (5), and ovarian cancer (5,6). Several investigators have attempted to characterize the putative neuronal antigens reacting with anti-Purkinje cell antibodies. Immunoblots with sera from PCD patients have identified several brain proteins of varying molecular weights (7-10), but the identity of these "target" proteins and their role in the pathogenesis of PCD remain unknown. Direct efforts at purifying the target proteins have been hampered by their restricted cellular distribution and relatively low abundance. We have used the autoantibodies from a patient with PCD to identify and clone a gene from a human cerebellum cDNA library. The gene is expressed predominantly in neuroectodermal tissues and codes for a protein with a distinctive tandemly repeating structure.

### Summary of the Invention

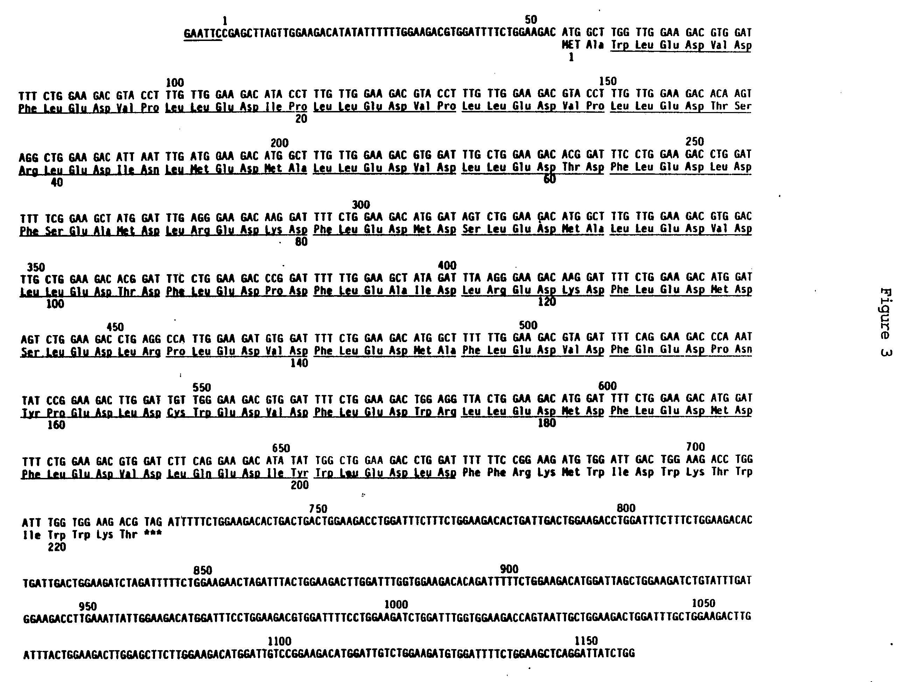

This invention provides a purified, naturally-occurring polypeptide comprising 222 amino acids having the sequence shown in Figure 3 beginning with alanine at the amino terminus and ending with threonine at the carboxy terminus.

This invention also provides a polypeptide comprising 223 amino acids having the sequence shown in Figure 3 beginning with methionine at the amino terminus and ending with threonine at the carboxy terminus. This invention further provides a nucleic acid molecule encoding the polypeptide.

This invention further provides a method of preventing paraneoplastic cerebellar degeneration in a subject susceptible to paraneoplastic cerebellar degeneration comprising administering to the subject an amount of a polypeptide of this invention effective to prevent paraneoplastic cerebellar degeneration.

Finally, this invention provides a method of preventing paraneoplastic cerebellar degeneration in a subject susceptible to paraneoplastic cerebellar degeneration comprising administering to the subject an amount of an antibody which specifically binds to a polypeptide of this invention effective to prevent paraneoplastic cerebellar degeneration.

### Brief Description of Figures

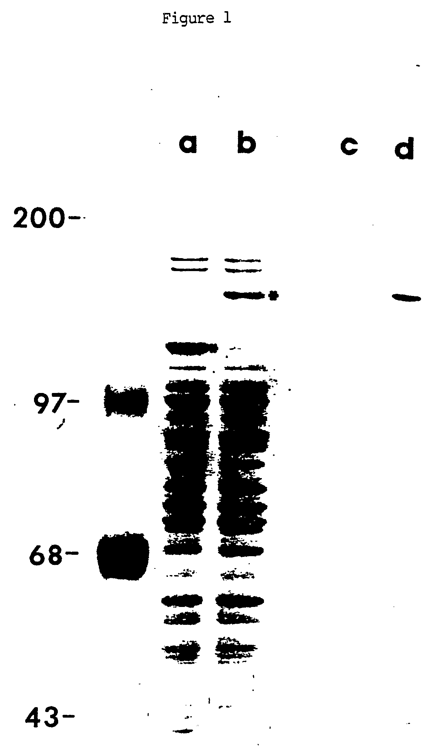

Figure 1 shows the characterization of the pCDR2 fusion protein. Lanes a and b, extracts of IPTG-induced lysogens from wild type lambda gt11 and PCDR2, respectively, were electrophoresed in 7% SDS-pAGE, and stained with Coomassie Blue. Bands at 116kD and 140kD in lanes a and b (asterisks) represent beta-galactosidase and the pCDR2 fusion protein, respectively. Lanes c and d, immunoblots of extracts from lanes a and b with the PCD patient's IgG, as visualized with the peroxidase technique. Molecular weight markers (kilodaltons) are indicated on the left.

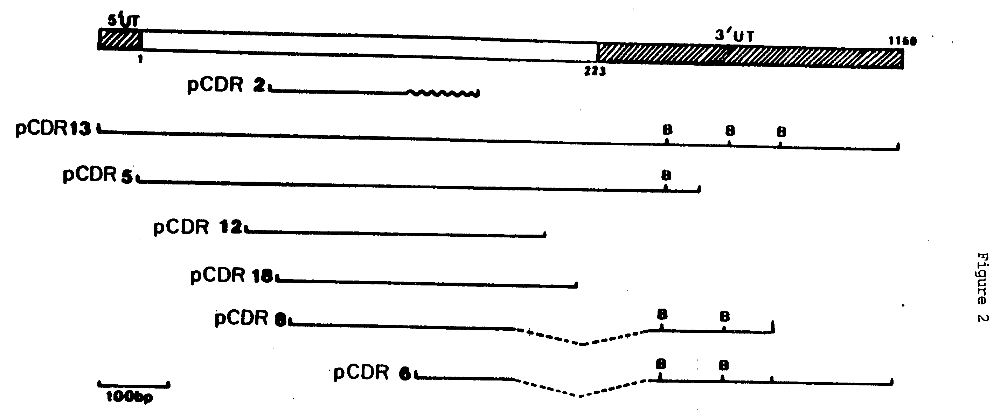

Figure 2 shows a restriction map of the CDR cDNA clones. Seven clones were aligned by DNA sequencing and by comparison of Bgl II restriction sites (B). Clones pCDR6 and pCDR8 lacked segments present in pCDR13 (dotted lines). pCDR2 contained a 120bp sequence in its 3' end (zigzag line) that was not present in the other clones and was proved to be a cloning artifact. Shaded areas in the composite sequence (top) represent 5' and 3' untranslated regions of pCDR13.

Figure 3 shows the nucleotide sequence and derived amino acid sequence of pCDR13 cDNA. The EcoR1 linker sequence is shown prior to nucleotide position 1. The numbers above and below the sequences are nucleotide and amino acid positions, respectively. The 18-nucleotide tandem repeating units are underlined. Asterisks indicate the translational termination codon.

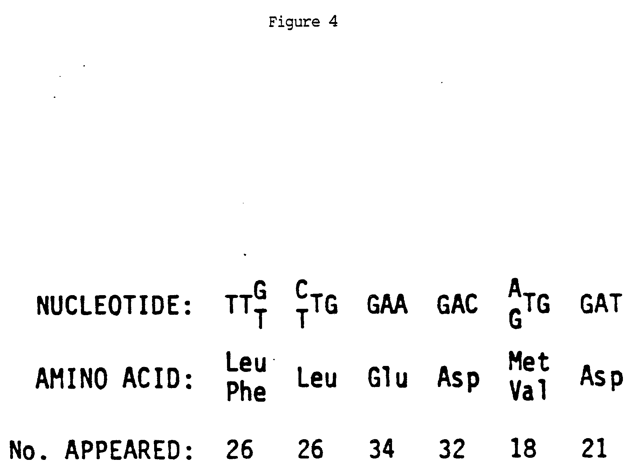

Figure 4 shows tandem repeats in the CDR sequence. The sequence of the 34 repeats is summarized to illustrate the consensus 18-nucleotide hexapeptide unit.

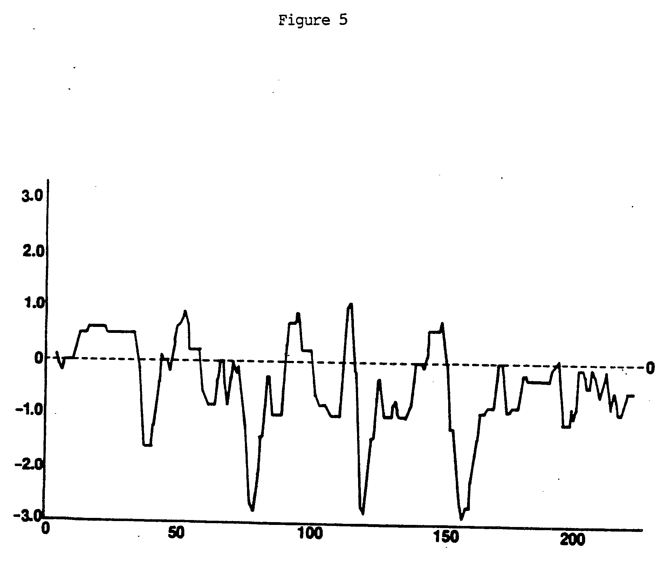

Figure 5 shows the hydropathy plot of the predicted amino acid sequence of the CDR protein based on the method of Kyte and Doolittle (23). Hydropathy values for a span of 7 amino acid residues were averaged, assigned to the middle residue of the span, and plotted (Y-axis) according to the position of the middle residue along the sequences (X-axis).

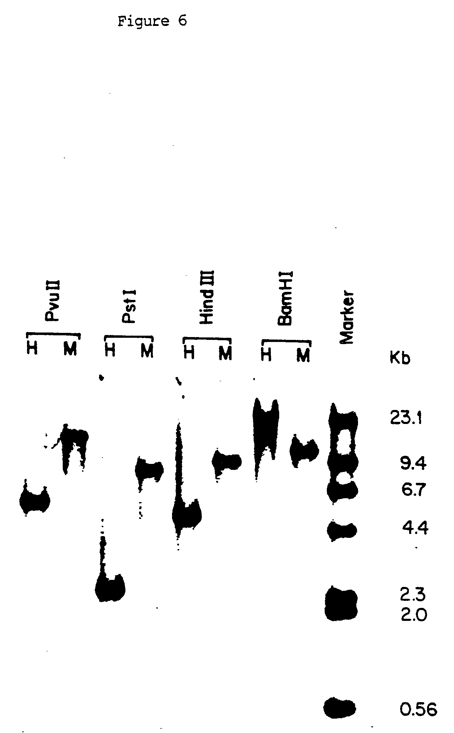

Figure 6 shows a southern blot analysis of genomic DNA fragments. Total cellular DNA was prepared from human (H) and mouse (M). DNA (15 micrograms per lane) was digested with restriction enzyme, electrophoresed through a 0.7% agarose gel, transferred to a nitrocellulose filter, and hybridized with $^{32}$P-labeled pCDR13 probe.

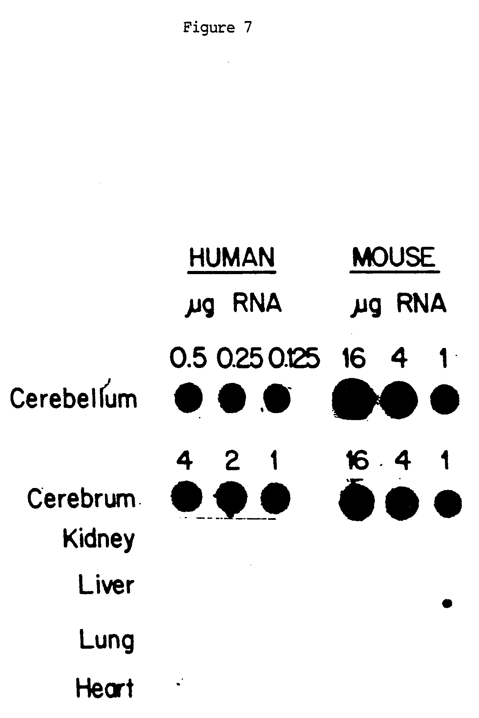

Figure 7 shows a dot blot hybridization of total cellular RNA from human and mouse tissues with pCDR13. Total cellular RNA was prepared from postmortem human tissues and BALB/c mouse tissues, transferred to nitrocellose filters, and hybridized with $^{32}$P-labeled nick-translated pCDR13.

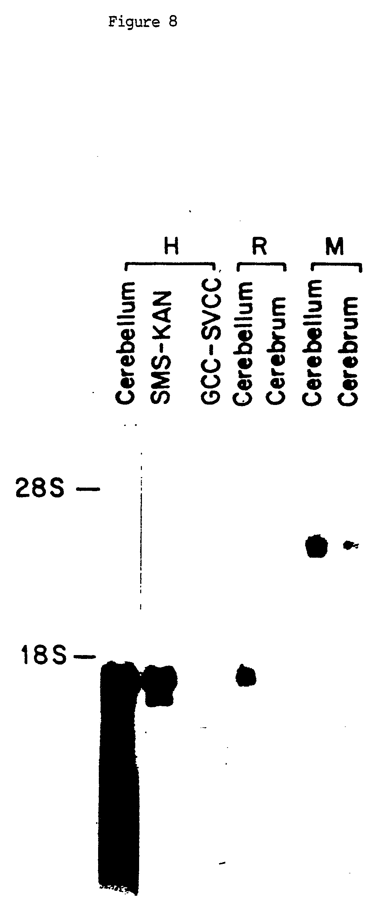

Figure 8 shows the northern blot hybridization of $^{32}$P-labeled pCDR13 with RNA from human (H), rabbit (R), and mouse (M) brain, neuroblastoma cell line SMS-KAN, and choriocarcinoma cell line GCC-SVCC. Amounts of RNA loaded: 5 micrograms total RNA from brain, 5 micrograms poly(A) + RNA from SMS-KAN and GCC-SVCC. Positions of 28S and 18S ribosomal RNA are indicated.

## Detailed Description of the Invention

This invention provides a purified, naturally occurring polypeptide comprising 222 amino acids having the amino acid sequence shown in Figure 3 beginning with alanine at the amino terminus and ending with threonine at the carboxy terminus.

This invention also provides a polypeptide comprising 223 amino acids having the sequence shown in Figure 3 beginning with methionine at the amino terminus and ending with threonine at the carboxy terminus. This invention also provides a nucleic acid molecule encoding this polypeptide. The nucleic acid molecule may be cDNA having the sequence shown in Figure 3 beginning at nucleotide 55 and ending with nucleotide 724.

This invention also provides a vector which comprises the nucleic acid molecule of this invention. This vector may be any vector known in the art. In a preferred embodiment the vector is a plasmid. Merely by way of example, the plasmid may be the plasmid designated pCPD13.

This invention also provides a host vector system for producing a polypeptide comprising 223 amino acids having the sequence shown in Figure 3. This host vector system comprises any vector known in the art which comprises the nucleic acid molecule of this invention and a suitable host. In a preferred embodiment, the vector is a plasmid. The host may include any host known in the art including a bacterial cell and a eucaryotic cell. Merely by way of example, the bacterial cell may be an E. coli cell and the eucaryotic cell may be an NIH 3T3 cell.

This invention further provides a method for producing a polypeptide comprising 223 amino acids having the amino acid sequence shown in Figure 3. The method comprises growing the host vector system of this invention so as to produce the polypeptide in the host and recovering the polypeptide produced.

This invention still further provides a pharmaceutical composition comprising an effective amount of the naturally occurring polypeptide comprising 222 amino acids having the sequence shown in Figure 3, or a fragment, thereof and pharmaceutically acceptable carrier. The pharmaceutically acceptable carrier may be any appropriate carrier known in the art. Merely by way of example, the carrier may be buffered saline.

This invention also provides a pharmaceutical composition comprising an effective amount of the polypeptide comprising 223 amino acids having the sequence shown in Figure 3, or a fragment thereof, and a pharmaceutically acceptable carrier. The pharmaceutically acceptable carrier may be any appropriate carrier known in the art. Merely by way of example, the carrier may be a buffered saline.

This invention also provides a method of preventing paraneoplastic cerebellar degeneration in a subject susceptible to paraneoplastic cerebellar degeneration. The method comprises administering to the subject an amount of a pharmaceutical composition comprising a polypeptide of this invention effective to prevent paraneoplastic cerebellar degeneration.

The amount of the polypeptide which is effective to prevent the occurrence of paraneoplastic cerebellar degeneration or prevent further development of paraneoplastic cerebellar degeneration depends on various factors. These factors include the progression of the disease causing the autoantibody production as well as the age, weight, and condition of the subject. However, in any subject an effective amount is an amount of polypeptide effective to cause a subject's autoantibodies to bind to the polypeptide and therefore prevent or minimize the binding of the autoantibodies to the cells in and around the subjects cerebellum.

Further, the pharmaceutical composition may be administered by any means which would allow interaction between the subject's autoantibodies and a portion of the polypeptide of this invention. The presently preferred means of administration is intravenous injection.

This invention further provides a noncytotoxic reagent which specifically binds to the naturally-occurring polypeptide comprising 222 amino acids or to the polypeptide comprising 223 amino acids both having the sequence shown in Figure 3. The noncytotoxic reagent may be an antibody and is preferably a monoclonal antibody.

This invention further provides a pharmaceutical composition comprising the antibody of this invention and a pharmaceutically acceptable carrier. This invention still further provides a method of preventing paraneoplastic cerebellar degeneration in a subject susceptible to paraneoplastic cerebellar degeneration comprising administering to the subject an amount of the pharmaceutical composition effective to prevent paraneoplastic cerebellar degeneration.

The amount of the antibody which is effective to prevent the occurrence of paraneoplastic cerebellar degeneration or prevent further development of paraneoplastic cerebellar degeneration depends on various factors including the progression of the disease causing the autoantibody production as well as the age, weight, and condition of the subject. The effective amount is an amount of antibody which is sufficient to effect binding of the antibodies to the cellular antigen occurring in or near the subjects cerebellum. The antibody-antigen complex prevents the binding of the subject's autoantibodies and thereby prevents paraneoplastic cerebellar degeneration.

The pharmaceutical composition may be administered by any means which would allow the antibodies of this invention to bind with the cellular antigens occurring in or near the subjects cerebellum. The presently preferred means of administration is intravenous injection.

While the methods described in this invention may potentially be used to treat animals their primary focus is on the prevention of paraneoplastic cerebellar degeneration in human beings. By susceptible, applicants contemplate any subject which has developed paraneoplastic cerebellar degeneration or is in a group of subjects which are at higher risk to develop paraneoplastic cerebellar degeneration. Currently, the subjects at higher risk to develop paraneoplastic cerebellar degeneration are subjects with neoplasms of the lung (especially small cell carcinoma), breast, ovary, or subjects with Hodgkins disease.

Materials and Methods

**Human and mouse tissue.** Normal human cerebellar cortex, cerebral cortex and other tissues were obtained at autopsy. BALB/c mice were from our breeding colony. Tumor cell lines were from the Sloan-Kettering Institute collection.

**DNA and RNA preparations.** High molecular weight cellular DNA and total cellular RNA were prepared as described (11). Poly(A)+ RNA was isolated by oligo (dT)-cellulose column chromatography (12).

**Construction of lambda gt11 cDNA library.** A lambda gt11 cDNA library was constructed from human cerebellar poly(A)+ RNA according to Huynh et al. (13). cDNA species larger than 300bp were size-selected by 5% polyacrylamide gel electrophoresis and ligated to EcoRI-digested, dephosphorylated lambda gt11 arms in a molar ratio of 2:1 (13,14). The ligated DNA was packaged using a high efficiency in vitro packaging extract (Vector Cloning Systems). Approximately $2.3 \times 10^5$ plaques were obtained, with 70% being recombinants. An amplified stock of the library was prepared in E. coli Y1088 (12) and used for antibody screening.

**Antibody screening of lambda gt11 library.** Serum used for screening was obtained from a patient with adenocarcinoma of unknown origin, PCD, an high-titer anti-Purkinje cell antibodies. IgG was isolated from the serum by ammonium sulfate precipitation and DEAE-Sephadex chromatography (15). Recombinant phage were screened at a density of 8000 plaque-forming units per 85 mm plate of E. coli Y1090. The plates were incubated at 42°C for 4 hours, overlaid with nitrocellulose filters (Millipore) saturated with IPTG, and incubated 3 hours at 37°C. Filters were washed in Tris/NaCl/Tween-20 and incubated at 4°C overnight with the primary antibody solution containing 2% bovine serum albumin and preabsorbed with a lysate of E. coli. Incubation at room temperature for 4 hours with horseradish peroxidase-conjugated goat anti-human IgG (BioRad) was followed by staining with chloronaphthol (BioRad). Clones yielding positive signals were purified by several rounds of antibody screening until 100% of the plaques gave positive signals.

**Preparation and immunoblotting of lambda gt11 fusion proteins.** Lysogens of recombinant phage were prepared in E. coli Y1089 (13) at a multiplicity of infection of five. Cultures were grown at 32°C to an OD550 = 0.5, followed by induction of lytic growth at 44°C for 30 min., addition of IPTG to a concentration of 10mM, and incubation at 37°C for 2 hrs. Bacterial pellets were lysed in cold Tris/EDTA/lysozyme buffer (16), sonicated, and centrifuged. The supernatants were diluted in SDS-PAGE sample buffer containing 5% 2-mercaptoethanol, electrophoresed in 7% SDS-PAGE (17), and electrophoretically transferred to nitrocellulose (18). Blots were analyzed with the patient's serum as described above for antibody screening.

**Plaque hybridization.** Nitrocellulose filters were overlaid on 85mm phage plates for 4 min, washed at room temperature for 4 min each in 1M NaCl/0.5N NaOH, 2M NaCl/1M Tris pH 6.8, and 2x SSC, then vacuum dried at 80°C for 2 hrs. Filters were hybridized and washed as described for Southern blot analysis (see below), except that dextran sulfate was omitted from the hybridization solution.

**DNA sequencing and analysis.** cDNA clones were subcloned into M13mp18 and M13mp19, sequenced by the dideoxy chain-termination method (19), and analyzed with computer programs from BIONET.

**Northern blot and cytoplasmic dot blot.** Northern blot and RNA dot blot analyses were done as described (20), using $^{32}$P-labeled nick-translated probes. For cytoplasmic dot blot analysis, cultured tumor cells were washed with phosphate-buffered saline, lysed in Tris EDTA/0.5% NP-40 containing 0.1mM vanadyl ribonucleoside complex (Bethesda Research Laboratory), and centrifuged (21). The supernatants were transferred to an equal volume of 12x SSC and 15% formaldehyde, bound to nitrocellulose paper in a Minifold apparatus (Schleicher and Schuell), and analyzed with nick-translated probes.

**Southern blot analysis.** High molecular weight genomic DNA (15 micrograms) was digested with restriction endonucleases, electrophoresed in 0.7% agarose gels and transferred to nitrocellulose (22). Filters were prehybridized and hybridized to nick-translated probes at 65°C for 15 hrs. Hybridization was performed in 5x SSC/4x Denhardt's solution/50mM phosphate buffer/50 micrograms/ml salmon sperm DNA/10% Dextran sulfate. Filters were then washed and autoradiographed.

## RESULTS

**Isolation of positive clones.** Initial antibody screening of 110,000 lambda gt11 recombinants from the human cerebellar cDNA library by the PCD patient's IgG yielded a single reproducibly positive clone, designated PcDR2. To confirm reactivity with antigenic determi nants encoded by the cDNA fusion protein, pCDR2 and wild type lambda gt11 were used to infect lysogenic strain Y1089. Bacterial lysates were prepared after induction of the phage beta-galactosidase gene by IPTG, and analyzed by SDS-PAGE (Fig. 1). The 116-kD beta-galactosidase present in the wild type culture was replaced by a 140kD fusion protein in the pCDR2 culture. Immunoblotting revealed that this fusion protein reacted specifically with the patient's IgG. No reaction was seen with IgG from normal individuals.

The pCDR2 clone was found to contain a 320bp cDNA insert, which was then used as a probe to rescreen the cerebellar cDNA library. Six positive overlapping clones were obtained (designated pCDR5,6,8,12,13, and 18) with inserts ranging in size from 500 to 1200 based pairs. None of the six clones identified by plaque hybridization produced a fusion protein which reacted with the patient's IgG. The pCDR13 clone was studied in detail, as it was the largest clone and contained overlapping sequences of the other six clones.

**Sequence analysis and repetitive features of CDR clones.** Figure 2 shows the restriction map of pCDR13 and the six other clones. Figure 3 shows the nucleotide and derived amino acid sequences of pDCR13. A continuous open reading frame commences with an ATG codon at position 55. This is the only long open reading frame present in the sequence, and is in register with the junctional lacZ sequence of lambda gt11. The 669 nucleotide open reading frame is followed by a 3' untranslated sequence. The protein encoded by the sequence from nucleotide positions 55 to 724 is comprised of 223 amino acids and has a calculated molecular weight of 27,038 daltons.

The most striking feature of the sequence is a pattern of tandem repeats consisting of units of 18 nucleotides throughout the entire cDNA sequence. The derived amino acid sequence contains 34 inexact tandem repeats of six amino acids that comprise 91% of the predicted peptide (Fig. 3 and 4). The hexapeptide repeating unit features a nearly invariant core of glutamic acid-aspartic acid (codons GAA GAC), flanked on the amino side by hydrophobic residues (generally leucine and phenylalamine) and on the carboxy side by an aliphatic residue, usually followed by aspartic acid. A nonrepetitive segment of 17 amino acids is found at the carboxy terminal of the peptide due to a single nucleotide insertion that disrupts the reading frame.

A hydropathy profile (23) of the predicted amino acid sequence (Fig. 5) reveals an initial hydrophobic segment, followed by cycles of alternating very hydrophilic and slightly hydrophobic regions with a periodicity of approximately 40 amino acid residues, and ending with a slightly hydrophilic nonrepetitive segment. The predicted secondary structure of the sequence according to the method of Chou and Fasman (24) is an alpha-helix, with a beta-sheet conformation extending along 30 amino acids at the carboxy end.

In comparison to the pCDR13 sequence, pCDR6 and PCDR8 both lack a 204bp segment that includes the translational termination codon of pCDR13. This indicates a possible alternate splicing of mRNA, as supported by the presence of multiple mRNA species in Northern blots (see below). Because of the deletion, the predicted amino acid sequences of pCDR6 and pCDR8 are slightly different from pCDR13 in the carboxy end of the mole cule, with the main repetitive feature remaining unchanged.

**Southern blot analysis of the CDR gene in human and mouse.** Southern blot analysis showed hybridization of the 1.2 kb pCDR13 probe to single fragments in digests of human genomic DNA by BamHI, EcoRI, Hind III, PstI, and PvuII (Fig. 6). These findings are consistent with single-copy representation of the CDR gene. The observation that different restriction enzymes generated single fragments also suggests that the entire pCDR13 cDNA clone was derived from one exon, or from exons lacking restriction sites in the intervening sequences. Southern analysis of restriction digits of mouse genomic DNA also showed strong hybridization signals (Fig. 6), indicating that the CDR gene is highly conserved between human and mouse.

**Analysis of CDR transcripts in normal and tumor cells.** Fig. 7 shows the RNA dot blot analysis of CDR transcripts in human and mouse tissue. CDR mRNA was detected in abundant amounts in human cerebellar cortex and cerebral hemisphere cortex, but was only barely detectable in lung, kidney, and heart muscle. At least 500-fold differences were estimated between the levels of CDR messages in brain and non-neural tissues. In mouse tissue, the mRNA was estimated to be 5 to 10 times more abundant in cerebellum than in cerebrum, and was undetectable in lung, heart, kidney, liver, and thymus.

Human tumor cell lines were also tested by cytoplasmic dot hybridization with the pCDR13 probe (Table 1).

6

## Table 1

### DETECTION OF CDR mRNA IN HUMAN TUMOR CELL LINES BY CYTOPLASMIC DOT BLOT ANLAYSIS

| Tumor Cell Lines | No. Positive/ No. tested | % positive |
|---|---|---|
| Neuroblastoma | 10/10 | 100 |
| Renal Cell carcinoma | 8/12 | 67 |
| Astrocytoma | 7/14 | 50 |
| Lung Carcinoma | 4/10 | 40 |
| Melanoma | 3/14 | 21 |
| Lymphoma/Leukemia | 0/9 | 0 |
| Colon Carcinoma | 0/10 | 0 |
| Breast Carcinoma | 0/8 | 0 |
| Other Carcinomas* | 0/6 | 0 |

*2 ovarian, 1 cervical, 1 bladder, 1 pancreatic, 1 choriocarcinoma

The message was present in all 10 neuroblastoma lines tested, as well as in the majority of renal cell carcinoma lines and a proportion of astrocytoma, melanoma, and lung carcinoma lines. The degree of expression by positive cell lines varied widely for a given tumor type, with no obvious correlation with morphological characteristics. The strongest expression was observed in several of the neuroblastoma and renal carcinoma lines, nearly approaching the abundance of the CDR message in normal brain tissue. The CDR message was not expressed in leukemia/lymphoma cell lines, or in lines derived from colon or breast carcinoma.

Northern blots of CDR mRNA-positive human cell lines demonstrated three closely migrating mRNA species (from 1.3kb to 1.5kb), best illustrated by the neuroblastoma line SMS-KAN (Fig. 8). This mRNA heterogeneity may be due to alternate splicing as suggested by structural studies of cDNA clones pCDR6 and pCDR8 (Fig. 3). Northern analysis of rabbit and mouse brain confirmed the greater expression of the CDR gene in cerebellum than in cerebrum (Fig. 8). Rabbit CDR messages were similar in size to the human transcripts, except for the absence of the 1.3kb species. In contrast, the blot of mouse brain RNA showed only a single 3.5kb message.

## Discussion

To identify the molecular targets for anti-brain antibodies in PCD, we have used the serum from a patient with PCD to isolate a cDNA clone from a human cerebellum lambda gt11 expression library. The nucleotide sequence of the cDR CDNA contains a pattern of tandem repeats with a structure that is unlike any known eukaryotic gene. In addition, the pattern of expression of the DNR gene does not resemble any previously described neuroeotodermal gene. CDR gene transcripts are found much more abundantly in normal adult brain, especially cerebellum, than in non-neural tissues. The low level of CDR message in human lung, kidney, and heart muscle could be due to expression in the small subpopulation of cells of neuroectodermal origin in these tissues.

Tandemly repeated sequences are known to characterize a number of surface proteins of malarial parasites. For example, the circumsporozoite protein of Plasmodium falciparum consists almost entirely of tandem repeats of 4 amino acids (25), while that of P. knowlesi contains 12 repeats of a 12-amino acid unit (26). These Plasmodium circumsporozoite proteins show a high degree of antigenicity and are able to serve as the targets for neutralizing antibodies. Most eukaryotic proteins with known tandem repeats are structural proteins, such as the intermediate filaments (27), nuclear lamins (28), myosin (29) and alpha-tropomyosin (30). These proteins form coiled coils and share a common structural pattern of tandemly repeating units of 7 amino acids, with hydrophobic residues occupying the first and fourth positions. However, there are otherwise no restrictions on the amino acids in the repeating units of these proteins, in contrast to the CDR molecule. Two proteins show certain similarities to the CDR gene product. Involucrin, a keratinocyte envelope component, contains a central segment of 39 repeats of 10 amino acids, and appears to be a substrate for cross-linking by transglutaminase (31). The PrP prion protein, thought to polymerize into fibrils, contains well-conserved tandem repeats of 8 amino acids extend ing over 13% of its sequence (32,33). To our knowledge, the only nonstructural eukaryotic protein with tandem repeats is RNA polymerase II, with a highly conserved unit of 7 amino acids repeated 26 times in the C-terminal sequence (34,35).

## References

1. Henson, R.A., Urich, H. (1982) Cancer and the Nervous System (Blackwell Scientific, Oxford).

2. Brain, L., Wilkinson, M. (1965) Brain 88, 465-478.

3. Trotter, J.L., Hendin, B.A., Osterland, C.K. (1976) Arch Neurol 33, 660-661.

4. Greenlee, J.E., Lipton, H.L. (1986) Ann Neurol 19, 82-85.

5. Jaeckle, K.A., Graus, F., Houghton, A.N., Cardon-Cordo, C., Nielsen, S., Posner, J. (1985) Ann Neurol, 18, 592-600.

6. Greenlee, J.E., Brashear, H.R. (1983) Ann Neurol 14, 609-613.

7. Brown, R.H., Ronthal, M., Come, S., Abend, W., Sotrel, A., Ogonowski, M., Weiner, H. (1985) Neurology 35 (suppl 1), 288.

8. Kornguth, S., Kalinke, T., Grunwald, G., Brooks, B., Zimmerman, E. (1985) Fed Proc 44, 966.

9. Tanaka, K., Yamazaki, M., Sato, S., Toyoshima, I., Yamamoto, A., Miyatake, T. (1986) Neurology 36, 1169-1172.

10. Cunningham, J., Graus, F., Anderson, N., Posner, J.B. (1986) Neurology 36, 1163-1168.

11. Obata, Y., Chen, Y.T., Stockert, E., Old, L.J. (1985) Proc Natl Acad Sci USA 82, 5475-5479.

12. Maniatis, T., Fritsch, E.F. & Sambrook, J. (1982) Molecular Cloning: A Laboratory Manual (Cold Spring Harbor Laboratory, Cold Spring Harbor, NY).

13. Suynh, T.V., Young, R.A., Davis, R.W. (1985) in DNA Cloning, ed. Glover, D.M. (IRL Press, Oxford), Vol. 1, pp. 49-78.

14. Young, R.A., Davis, R.W. (1983) Proc Natl Acad Sci USA 80, 1194-1198.

15. Mishell, B.B., Shiigi, S.M., eds. (1980) in Selected Methods in Celllar Immunology (W.H. Freeman & Co., San Francisco).

16. Gallin, W.J., Prediger, E.A., Edelman, G.M., Cunningham, B.A. (1985) Proc Natl Acad Sci USA 82, 2809-2813.

17. Laemmli, U.K. (1970) Nature 227, 680-685.

18. Towbin, H., Staehelin, T., Gordon, J. (1979) Proc Natl Acad Sci USA 76, 4350-4354.

19. Sanger, F., Nicklen, S., Coulson, A.R. (1977) Proc Natl Acad Sci USA 74, 5463-5467.

20. Chen, Y.T., Obata, Y., Stockert, E., Old, L.J. (1985) J Esp Med 162, 1134-1148.

21. White, B.A., Bancroft, F.C. (1982) J Biol Chem 257, 8569-8572.

22. Southern, E.M. (1975) J Mol Biol 98, 503-517.

23. Kyte, J., Doolittle, R.F. (1982) J Mol Biol 98, 503-517.

24. Chou, P.Y., Fasman, G.D. (1978) Ann Rev Biochem 47, 251-276.

25. Dame, J.B., Williams, J.L., McCutchon, T.F., Weber, J.L. (1984) Science 225, 593-599.

26. Ellis, J., Ozaki, L.S., Gwadz, R.W., Cochrane, A.H. (1983) Nature 302, 536-538.

27. Marchule, D., McCroham, S., Fuchs, E. (1984) Cell 39, 491-498.

28. Fisher, O.Z., Chaudhary, N., Blobel, G. (1986) Proc Natl Acad Sci USA 83, 6450-6454.

29. DeLayanne, A., Lewis, M., Spudich, J.A., Lernuirand, L.A. (1985) Proc Natl Acad Sci USA 82, 6707-6810.

30. Stone, D., Smillie, L.B. (1978) J Biol Chem 253, 1137-1148.

31. Eckert, R.L., Green, H. (1986) Cell 46, 583-589.

32. Locht, C., Chesebro, B., Race, R., Keith, J.M. (1986) Proc Natl Acad Sci USA 83, 6372-6376.

33. Oesch, B., Westaway, D., Walchli, M., McKinley, M.P. (1985) Cell 40, 735-736.

34. Allison, L.A., Mayle, M., Shales, M., Ingles, C.J. (1985) Cell 42, 599-610.

35. Corden, J.L., Cadena, D.L., Ahearn, J.M., Dahmus, M.E. (1985) Proc Natl Acad Sci USA 82, 7934-7938.

## Claims

1. A purified, naturally-occurring polypeptide comprising 222 amino acids having the sequence shown in Figure 3 beginning with alanine at the amino terminus and ending with threonine at the carboxy terminus.

2. A polypeptide comprising 223 amino acids having the sequence shown in Figure 3 beginning with methionine at the amino terminus and ending with threonine at the carboxy terminus.

3. A nucleic acid molecule encoding the polypeptide of claim 2.

4. A cDNA of claim 3 having the sequence shown in Figure 3 beginning at nucleotide 55 and ending with nucleotide 724.

5. A plasmid which comprises the nucleic acid molecule of claim 3.

6. A host vector system for producing the polypeptide comprising 223 amino acids having the sequence shown in Figure 3, comprising a plasmid of claim 5 in a suitable host.

7. A host vector system of claim 6, wherein the suitable host is a bacteria cell.

8. A host vector system of claim 6, wherein the suitable host is a eucaryotic cell.

9. A method for producing a polypeptide comprising 223 amino acids having the sequence shown in Figure 3, comprising growing the host vector system of claim 6 so as to produce the polypeptide in the host and recovering the polypeptide so produced.

10. A pharmaceutical composition comprising an effective amount of the polypeptide of claim 1 or 2 or a fragment thereof and a pharmaceutically acceptable carrier.

11. A method of preventing paraneoplastic cerebellar degeneration in a subject susceptible to paraneoplastic cerebellar degeneration comprising administering to the subject an amount of the composition of claim 10 effective to prevent paraneoplastic cerebellar degeneration.

12. A method of claim 11 wherein the subject is a human being.

13. A method of claim 11 wherein the polypeptide or fragment thereof is administered intravenously.

14. A noncytotoxic reagent which specifically binds to the polypeptide of claim 1 or 2.

15. An antibody of claim 14.

16. A monoclonal antibody of claim 15.

17. A pharmaceutical composition comprising the antibody of claim 15 and a pharmaceutically acceptable carrier.

18. A method of preventing paraneoplastic cerebellar degeneration in a subject susceptible to paraneoplastic cerebellar degeneration comprising administering to the subject an amount of the composition of claim 17 effective to prevent paraneoplastic cerebellar degeneration.

19. A method of claim 18, wherein the subject is a human being.

20. A method of claim 18, wherein the antibody is administered intravenously.

Figure 1

Figure 2

EP 0 297 585 A2

```
        1                                                    50
GAATTCCGAGCTTAGTTGGAAGACATATATTTTTTGGAAGACGTGGATTTTCTGGAAGAC ATG GCT TGG TTG GAA GAC GTG GAT
                                                             MET Ala Trp Leu Glu Asp Val Asp
                                                                 1


                   100                                              150
TTT CTG GAA GAC GTA CCT TTG TTG GAA GAC ATA CCT TTG TTG GAA GAC GTA CCT TTG TTG GAA GAC GTA CCT TTG TTG GAA GAC ACA AGT
Phe Leu Glu Asp Val Pro Leu Leu Glu Asp Ile Pro Leu Leu Glu Asp Val Pro Leu Leu Glu Asp Val Pro Leu Leu Glu Asp Thr Ser
                                                  20


                   200                                              250
AGG CTG GAA GAC ATT AAT TTG ATG GAA GAC ATG GCT TTG TTG GAA GAC GTG GAT TTG CTG GAA GAC ACG GAT TTC CTG GAA GAC CTG GAT
Arg Leu Glu Asp Ile Asn Leu Met Glu Asp Met Ala Leu Leu Glu Asp Val Asp Leu Leu Glu Asp Thr Asp Phe Leu Glu Asp Leu Asp
    40                                                            60


                        300
TTT TCG GAA GCT ATG GAT TTG AGG GAA GAC AAG GAT TTT CTG GAA GAC ATG GAT AGT CTG GAA GAC ATG GCT TTG TTG GAA GAC GTG GAC
Phe Ser Glu Ala Met Asp Leu Arg Glu Asp Lys Asp Phe Leu Glu Asp Met Asp Ser Leu Glu Asp Met Ala Leu Leu Glu Asp Val Asp
                          80


  350                                       400
TTG CTG GAA GAC ACG GAT TTC CTG GAA GAC CCG GAT TTT TTG GAA GCT ATA GAT TTA AGG GAA GAC AAG GAT TTT CTG GAA GAC ATG GAT
Leu Leu Glu Asp Thr Asp Phe Leu Glu Asp Pro Asp Phe Leu Glu Ala Ile Asp Leu Arg Glu Asp Lys Asp Phe Leu Glu Asp Met Asp
    100                                                          120


          450                                    500
AGT CTG GAA GAC CTG AGG CCA TTG GAA GAT GTG GAT TTT CTG GAA GAC ATG GCT TTT TTG GAA GAC GTA GAT TTT CAG GAA GAC CCA AAT
Ser Leu Glu Asp Leu Arg Pro Leu Glu Asp Val Asp Phe Leu Glu Asp Met Ala Phe Leu Glu Asp Val Asp Phe Gln Glu Asp Pro Asn
                                            140


              550                                      600
TAT CCG GAA GAC TTG GAT TGT TGG GAA GAC GTG GAT TTT CTG GAA GAC TGG AGG TTA CTG GAA GAC ATG GAT TTT CTG GAA GAC ATG GAT
Tyr Pro Glu Asp Leu Asp Cys Trp Glu Asp Val Asp Phe Leu Glu Asp Trp Arg Leu Leu Glu Asp Met Asp Phe Leu Glu Asp Met Asp
    160                                                    180


                        650                                            700
TTT CTG GAA GAC GTG GAT CTT CAG GAA GAC ATA TAT TGG CTG GAA GAC CTG GAT TTT TTC CGG AAG ATG TGG ATT GAC TGG AAG ACC TGG
Phe Leu Glu Asp Val Asp Leu Gln Glu Asp Ile Tyr Trp Leu Glu Asp Leu Asp Phe Phe Arg Lys Met Trp Ile Asp Trp Lys Thr Trp
                                            200


                    750                                              800
ATT TGG TGG AAG ACG TAG ATTTTTCTGGAAGACACTGACTGACTGGAAGACCTGGATTTCTTTCTGGAAGACACTGATTGACTGGAAGACCTGGATTTCTTTCTGGAAGACAC
Ile Trp Trp Lys Thr ***
    220


                  850                                      900
TGATTGACTGGAAGATCTAGATTTTTCTGGAAGAACTAGATTTACTGGAAGACTTGGATTTGGTGGAAGACACAGATTTTTCTGGAAGACATGGATTAGCTGGAAGATCTGTATTTGAT

        950                                   1000                                       1050
GGAAGACCTTGAAATTATTGGAAGACATGGATTTCCTGGAAGACGTGGATTTTTCCTGGAAGATCTGGATTTGGTGGAAGACCAGTAATTGCTGGAAGACTGGATTTGCTGGAAGACTTG

                     1100                                   1150
ATTTACTGGAAGACTTGGAGCTTCTTGGAAGACATGGATTGTCCGGAAGACATGGATTGTCTGGAAGATGTGGATTTTCTGGAAGCTCAGGATTATCTGG
```

Figure 3

Figure 4

NUCLEOTIDE:  TT$^G_T$  $^C_T$TG  GAA  GAC  $^A_G$TG  GAT

AMINO ACID:  $^{Leu}_{Phe}$  Leu  Glu  Asp  $^{Met}_{Val}$  Asp

No. APPEARED:  26    26    34    32    18    21

Figure 5

Figure 6

Figure 7

|  | HUMAN μg RNA | | | MOUSE μg RNA | | |
|---|---|---|---|---|---|---|
|  | 0.5 | 0.25 | 0.125 | 16 | 4 | 1 |
| Cerebellum | ● | ● | ● | ● | ● | ● |
|  | 4 | 2 | 1 | 16 | 4 | 1 |
| Cerebrum | ● | ● | ● | ● | ● | ● |
| Kidney |  |  |  |  |  |  |
| Liver |  |  |  |  |  | ● |
| Lung |  |  |  |  |  |  |
| Heart |  |  |  |  |  |  |

Figure 8